# EUROPEAN PATENT APPLICATION

(11) **EP 4 449 992 A1**
(43) Date of publication of application: **23.10.2024**
(21) Application number: 22922122.1
(22) Date of filing: 12.12.2022
(51) Int. Cl.: A61B 6/00, A61B 6/02

(54) **IMAGE PROCESSING DEVICE, IMAGE PROCESSING METHOD, PROGRAM, AND MACHINE LEARNING METHOD**

(30) Priority: 19.01.2022 JP 2022006672
(71) Applicant: FUJIFILM Corporation, Tokyo 106-8620 (JP)
(72) Inventor: MACHII, Yusuke, Ashigarakami-gun, Kanagawa 258-8538 (JP)
(74) Representative: Dehns Germany Partnerschaft mbB
(86) International application number: PCT/JP2022/045734
(87) International publication number: WO 2023/139971

(57) **Abstract**

There is provided an image processing apparatus that executes calcification image detection processing of detecting a calcification image based on a plurality of tomographic images obtained from a series of a plurality of projection images obtained by tomosynthesis imaging of a breast; region-of-interest image generation processing of generating a region-of-interest image based on a detection result by the calcification image detection processing by cutting out a region including the calcification image, from a projection image obtained at an irradiation position among the plurality of projection images or the plurality of tomographic images, the irradiation position being closest to a position facing a detection surface of a radiation detector; shape restoration processing of restoring a shape of the calcification image based on the region-of-interest image generated by the region-of-interest image generation processing; and synthesized two-dimensional image generation processing of generating a synthesized two-dimensional image based on a shape restoration result by the shape restoration processing and the plurality of tomographic images.

## Description

### BACKGROUND OF THE INVENTION

### 1. Field of the Invention

The present disclosure relates to an image processing apparatus, an image processing method, a program, and a machine learning method.

### 2. Description of the Related Art

A technology for recognizing a tissue of a breast in which calcification may occur by using a radiation image obtained by irradiating the breast with radiations is known. For example, JP2020-127669A discloses a technique of specifying a region of a subject on a radiation image based on a position of calcification on volume data of the subject and generating a highlighted image in which the position of the calcification in the radiation image is highlighted. Further, JP2020-127669A discloses a technique of highlighting and displaying a position of calcification or a shape and a property of the calcification on the radiation image using a method such as colors.

In addition, tomosynthesis imaging in which a series of a plurality of projection images is acquired by irradiating a breast with radiations having a plurality of angles is known. By reconfiguring the plurality of projection images obtained by tomosynthesis imaging, a plurality of tomographic images in which an overlap of mammary glands is reduced are obtained. Further, a technique of generating one synthesized two-dimensional image in which an overlap of mammary glands is reduced by synthesizing a plurality of tomographic images is known. In addition, JP2020-141867A discloses a technique of generating a two-dimensional image corresponding to the synthesized two-dimensional image by inputting a projection image obtained at a radiation irradiation angle of approximately 0 degree to a learned model instead of the plurality of tomographic images.

### SUMMARY OF THE INVENTION

In image diagnosis for diagnosing calcification of a breast, a shape of a calcification image appearing in a tomographic image, a synthesized two-dimensional image, or the like is important information. However, in the tomographic image, the calcification image is blurred due to noise and visibility is lowered. In addition, in the synthesized two-dimensional image generated based on the plurality of tomographic images, a shape of the calcification image is not accurately represented.

JP2020-127669A discloses a technique of performing highlight display based on a shape of a calcification image. However, the technique is not a technique for improving visibility of a shape of a calcification image. In addition, JP2020-141867A discloses a technique of generating a synthesized two-dimensional image. However, there is no description of improving visibility of a shape of a calcification image in a synthesized two-dimensional image.

In this way, a shape of a calcification image in a synthesized two-dimensional image is deteriorated as compared with a calcification image in a projection image acquired by plain radiography. For this reason, it is desired to restore a shape of a calcification image in a synthesized two-dimensional image into a shape of a calcification image in a projection image acquired by plain radiography.

An object of the technology of the present disclosure is to provide an image processing apparatus, an image processing method, a program, and a machine learning method capable of improving visibility of a shape of a calcification image in a synthesized two-dimensional image.

In order to achieve the above object, according to the present disclosure, there is provided an image processing apparatus including: at least one processor, in which the processor is configured to execute: calcification image detection processing of detecting a calcification image based on a plurality of tomographic images obtained from a series of a plurality of projection images obtained by tomosynthesis imaging of a breast; region-of-interest image generation processing of generating a region-of-interest image based on a detection result by the calcification image detection processing by cutting out a region including the calcification image, from a projection image obtained at an irradiation position among the plurality of projection images or the plurality of tomographic images, the irradiation position being closest to a position facing a detection surface of a radiation detector; shape restoration processing of restoring a shape of the calcification image based on the region-of-interest image generated by the region-of-interest image generation processing; and synthesized two-dimensional image generation processing of generating a synthesized two-dimensional image based on a shape restoration result by the shape restoration processing and the plurality of tomographic images.

Preferably, the processor is configured to: in a case where a plurality of calcification images are detected in the calcification image detection processing, individually generate the region-of-interest image for each of the plurality of calcification images in the region-of-interest image generation processing.

Preferably, the processor is configured to: execute the shape restoration processing by inputting the region-of-interest image into a machine-learned model.

Preferably, the machine-learned model is a neural network obtained by performing machine learning by using, as an input image, the region-of-interest image and using, as a correct answer image, an image generated by cutting out a region including the calcification image from a projection image obtained by plain radiography in which radiation is emitted from a position facing a detection surface of a radiation detector.

Preferably, the machine-learned model is a neural network obtained by performing machine learning by using an input image and a correct answer image that are generated by a simulation or by imaging using a phantom.

According to the present disclosure, there is provided an image processing method including: a calcification image detection step of detecting a calcification image based on a plurality of tomographic images obtained from a series of a plurality of projection images obtained by tomosynthesis imaging of a breast; a region-of-interest image generation step of generating a region-of-interest image based on a detection result by the calcification image detection step by cutting out a region including the calcification image, from a projection image obtained at an irradiation position among the plurality of projection images or the plurality of tomographic images, the irradiation position being closest to a position facing a detection surface of a radiation detector; a shape restoration step of restoring a shape of the calcification image based on the region-of-interest image generated by the region-of-interest image generation step; and a synthesized two-dimensional image generation step of generating a synthesized two-dimensional image based on a shape restoration result by the shape restoration step and the plurality of tomographic images.

According to the present disclosure, there is provided a program causing a computer to execute: calcification image detection processing of detecting a calcification image based on a plurality of tomographic images obtained from a series of a plurality of projection images obtained by tomosynthesis imaging of a breast; region-of-interest image generation processing of generating a region-of-interest image based on a detection result by the calcification image detection processing by cutting out a region including the calcification image, from a projection image obtained at an irradiation position among the plurality of projection images or the plurality of tomographic images, the irradiation position being closest to a position facing a detection surface of a radiation detector; shape restoration processing of restoring a shape of the calcification image based on the region-of-interest image generated by the region-of-interest image generation processing; and synthesized two-dimensional image generation processing of generating a synthesized two-dimensional image based on a shape restoration result by the shape restoration processing and the plurality of tomographic images.

According to the present disclosure, there is provided a machine learning method including: detecting a calcification image based on a plurality of tomographic images obtained from a series of a plurality of projection images obtained by tomosynthesis imaging of a breast; setting, as an input image, a region-of-interest image generated by cutting out a region including the calcification image, from a projection image obtained at an irradiation position among the plurality of projection images or the plurality of tomographic images, the irradiation position being closest to a position facing a detection surface of a radiation detector; and performing machine learning on a neural network by using, as a correct answer image, an image generated by cutting out a region including the calcification image from a projection image obtained by plain radiography in which radiation is emitted from a position facing a detection surface of a radiation detector.

According to the technology of the present disclosure, it is possible to provide an image processing apparatus, an image processing method, a program, and a machine learning method capable of improving visibility of a shape of a calcification image in a synthesized two-dimensional image.

### BRIEF DESCRIPTION OF THE DRAWINGS

Fig. 1 is a diagram illustrating an example of an entire configuration of a radiography system.
Fig. 2 is a diagram illustrating an example of tomosynthesis imaging.
Fig. 3 is a block diagram illustrating an example of a configuration of an image processing apparatus.
Fig. 4 is a block diagram illustrating an example of a function realized by a control unit of the image processing apparatus.
Fig. 5 is a diagram schematically illustrating a flow of processing by the image processing apparatus.
Fig. 6 is a diagram conceptually illustrating an example of region-of-interest image generation processing.
Fig. 7 is a diagram conceptually illustrating an example of synthesized two-dimensional image generation processing.
Fig. 8 is a flowchart illustrating a flow of a series of processing by the image processing apparatus.
Fig. 9 is a diagram conceptually illustrating an example of learning processing in a learning phase.
Fig. 10 is a block diagram illustrating a function realized by a control unit of the image processing apparatus according to a first modification example.
Fig. 11 is a diagram conceptually illustrating learning processing according to a first modification example.
Fig. 12 is a diagram illustrating an example of generating training data by simulation.

### DESCRIPTION OF THE PREFERRED EMBODIMENTS

Hereinafter, an embodiment of the present disclosure will be described in detail with reference to the drawings.

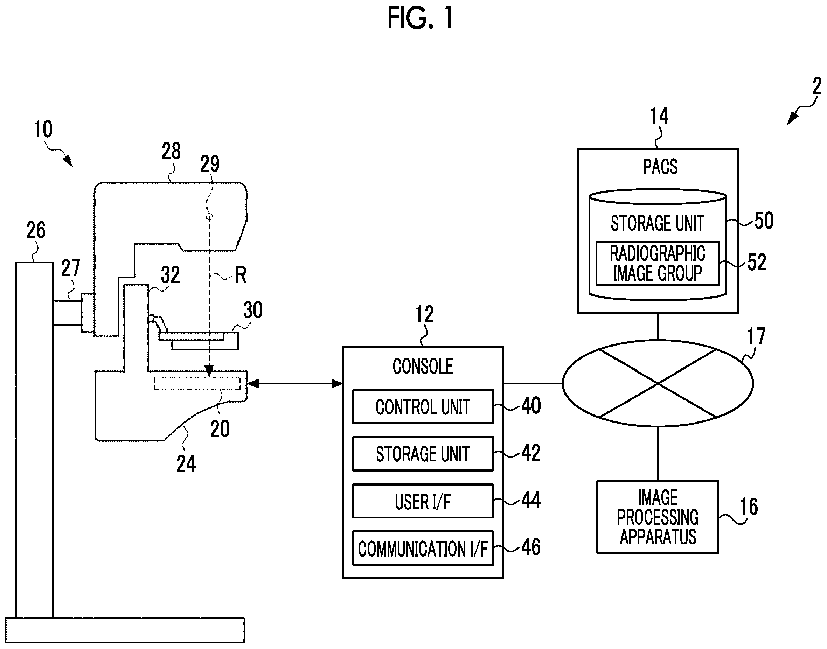

Fig. 1 illustrates an example of an entire configuration of a radiography system 2 according to the present embodiment. The radiography system 2 includes a mammography apparatus 10, a console 12, a picture archiving and communication systems (PACS) 14, and an image processing apparatus 16. The console 12, the PACS 14, and the image processing apparatus 16 are connected to each other via a network 17 by wired communication or wireless communication.

Fig. 1 illustrates an example of an appearance of the mammography apparatus 10. Fig. 1 illustrates an example of an appearance in a case where the mammography apparatus 10 is viewed from a left side of a subject.

The mammography apparatus 10 operates according to a control of the console 12, and is a radiographic imaging apparatus that acquires a radiation image of a breast M by irradiating the breast M of the subject as a target with radiations R (for example, X rays) from a radiation source 29.

The mammography apparatus 10 has a function of performing plain radiography in which imaging is performed in a state where the radiation source 29 is positioned at an irradiation position along a normal direction of a detection surface 20A of a radiation detector 20 and a function of performing tomosynthesis imaging in which imaging is performed in a state where the radiation source 29 is moved to each of a plurality of irradiation positions.

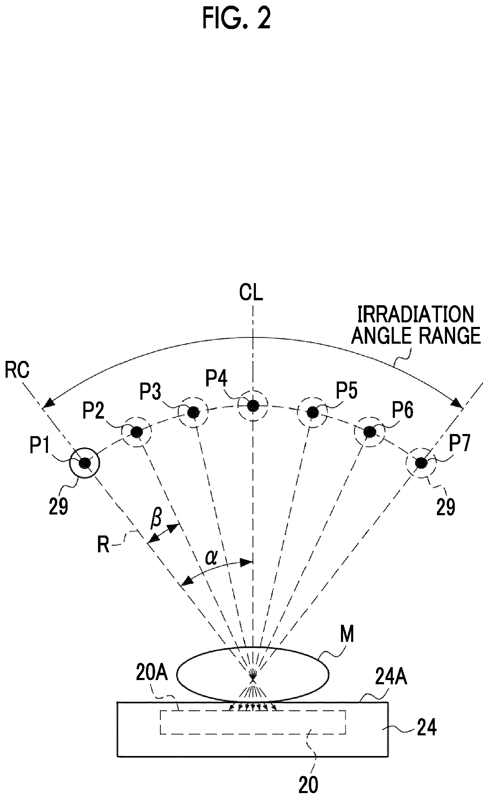

As illustrated in Fig. 1, the mammography apparatus 10 includes an imaging table 24, a base 26, an arm portion 28, and a compression unit 32. A radiation detector 20 is disposed inside the imaging table 24. As illustrated in Fig. 2, in the mammography apparatus 10, in a case of performing imaging, the breast M of the subject is positioned on an imaging surface 24A of the imaging table 24 by a user.

The radiation detector 20 detects radiations R passing through the breast M as a target. Specifically, the radiation detector 20 detects the radiations R that pass through the breast M of the subject, enter into the imaging table 24, and reach a detection surface 20A of the radiation detector 20, and generates a radiation image based on the detected radiations R. The radiation detector 20 outputs image data representing the generated radiation image. In the following, a series of operations of irradiating the breast with radiations R from the radiation source 29 and generating a radiation image by the radiation detector 20 may be referred to as "imaging". The radiation detector 20 may be an indirect-conversion-type radiation detector that converts the radiations R into light beams and converts the converted light beams into charges, or may be a direct-conversion-type radiation detector that directly converts the radiations R into charges.

A compression plate 30 that is used for compressing the breast M when performing imaging is attached to the compression unit 32. The compression plate 30 is moved in a direction toward or away from the imaging table 24 (hereinafter, referred to as a "vertical direction") by a compression plate driving unit (not illustrated) provided in the compression unit 32. The compression plate 30 compresses the breast M between the compression plate 30 and the imaging table 24 by moving in the vertical direction.

The arm portion 28 can be rotated with respect to the base 26 by a shaft portion 27. The shaft portion 27 is fixed to the base 26, and the shaft portion 27 and the arm portion 28 are rotated as one body. Gears are provided in each of the shaft portion 27 and the compression unit 32 of the imaging table 24. By switching the gears between an engaged state and a non-engaged state, the compression unit 32 of the imaging table 24 and the shaft portion 27 can be switched between a state where the compression unit 32 and the shaft portion 27 are connected to each other and are rotated as one body and a state where the shaft portion 27 is separated from the imaging table 24 and idles. Elements for switching between transmission and non-transmission of power of the shaft portion 27 are not limited to the gears, and various mechanical elements can be used. The arm portion 28 and the imaging table 24 can be separately rotated with respect to the base 26 with the shaft portion 27 as a rotation axis.

In a case of performing tomosynthesis imaging in the mammography apparatus 10, the radiation source 29 is sequentially moved to each of a plurality of irradiation positions having different irradiation angles by rotation of the arm portion 28. The radiation source 29 includes a radiation tube (not illustrated) that generates the radiations R, and the radiation tube is moved to each of the plurality of irradiation positions in accordance with the movement of the radiation source 29.

Fig. 2 illustrates an example of tomosynthesis imaging. In Fig. 2, the compression plate 30 is not illustrated. In the present embodiment, the radiation source 29 is moved to irradiation positions Pk (k = 1, 2, ..., 7) at which irradiation angles are different by a certain angle β. That is, the radiation source 29 is sequentially moved to a plurality of positions at which the irradiation angles of the radiations R with respect to the detection surface 20A of the radiation detector 20 are different. In Fig. 2, the number of the irradiation positions Pk is set to 7. On the other hand, the number of the irradiation positions Pk is not limited and can be changed as appropriate.

At each irradiation position Pk, the radiation R is emitted from the radiation source 29 toward the breast M, and the radiation detector 20 generates a radiation image by detecting the radiation R passing through the breast M. In the radiography system 2, in a case where the radiation source 29 is moved to each of the irradiation positions Pk and tomosynthesis imaging for generating a radiation image at each irradiation position Pk is performed, in the example of Fig. 2, seven radiation images are obtained.

In the following, in the tomosynthesis imaging, the radiation image obtained by performing imaging at each irradiation position Pk is referred to as a "projection image" in a case of distinguishing and describing the radiation image from a tomographic image, and a plurality of projection images obtained by performing tomosynthesis imaging once are referred to as a "series of the plurality of projection images". Further, in a case where the projection image is referred to without distinguishing the projection image from the tomographic image, the projection image is simply referred to as a "radiation image".

In addition, as illustrated in Fig. 2, the irradiation angle of the radiation R means an angle α formed by a normal line CL of the detection surface 20A of the radiation detector 20 and a radiation axis RC. The radiation axis RC means an axis connecting a focus of the radiation source 29 at each irradiation position Pk and a preset position. Further, the detection surface 20A of the radiation detector 20 is a surface substantially parallel to the imaging surface 24A. The radiation R emitted from the radiation source 29 is a cone beam having a focus as the apex and the radiation axis RC as a central axis.

On the other hand, in a case of performing plain radiography in the mammography apparatus 10, the position of the radiation source 29 is fixed to the irradiation position P4 at which the irradiation angle α is 0 degree. The radiation R is emitted from the radiation source 29 according to an instruction of the console 12, and the radiation detector 20 generates a radiation image by detecting the radiation R passing through the breast M. For example, in the plain radiography, the radiation R is emitted from the radiation source 29 with a higher radiation dose than that in the tomosynthesis imaging.

The mammography apparatus 10 and the console 12 are connected to each other by wired communication or wireless communication. The radiation image generated by the radiation detector 20 in the mammography apparatus 10 is output to the console 12 by wired communication or wireless communication via a communication interface (I/F) (not illustrated).

The console 12 includes a control unit 40, a storage unit 42, a user I/F 44, and a communication I/F 46. As described above, the control unit 40 has a function of performing control related to radiographic imaging by the mammography apparatus 10. The control unit 40 is configured with, for example, a computer system including a central processing unit (CPU), a read only memory (ROM), and a random access memory (RAM).

The storage unit 42 stores information related to radiographic imaging, the radiation image acquired from the mammography apparatus 10, and the like. The storage unit 42 is a non-volatile storage such as a hard disk drive (HDD) or a solid state drive (SSD).

The user I/F 44 includes an input device including various buttons and switches, which are related to imaging of the radiation image and are operated by a user such as a technician, and a display device such as a lamp or a display that displays information related to imaging, the radiation image obtained by imaging, and the like.

The communication I/F 46 performs communication of various types of data such as the information related to radiographic imaging, the radiation image, and the like between the console 12 and the mammography apparatus 10 by wired communication or wireless communication. Further, the communication I/F 46 performs communication of various types of data such as the radiation image between the PACS 14 and the image processing apparatus 16 via the network 17 by wired communication or wireless communication.

In addition, the PACS 14 includes a storage unit 50 (refer to Fig. 1) that stores a radiation image group 52. The radiation image group 52 includes a projection image acquired from the console 12 via the network 17.

The image processing apparatus 16 has a function of supporting diagnosis by a doctor by performing determination related to diagnosis of a lesion in a case where a doctor or the like (hereinafter, simply referred to as a "doctor") performs diagnosis related to a lesion of the breast M using the radiation image.

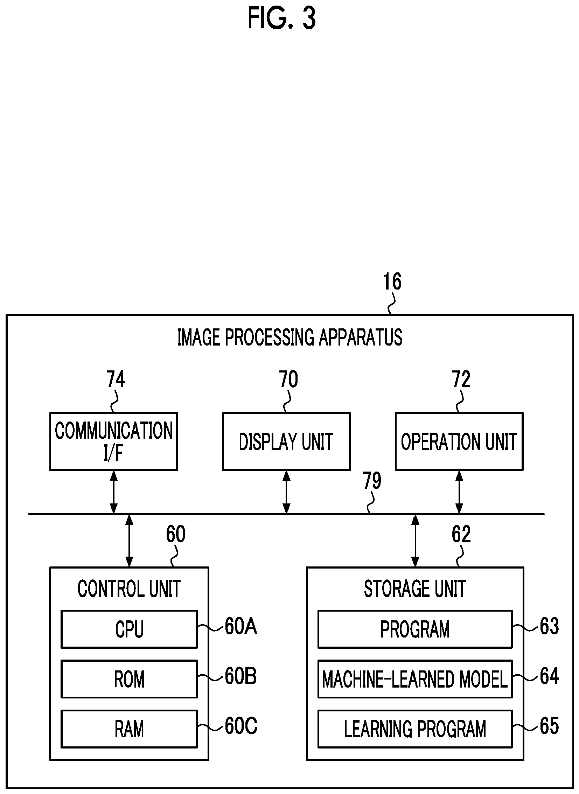

Fig. 3 illustrates an example of a configuration of the image processing apparatus 16. The image processing apparatus 16 includes a control unit 60, a storage unit 62, a display unit 70, an operation unit 72, and a communication I/F 74. The control unit 60, the storage unit 62, the display unit 70, the operation unit 72, and the communication I/F 74 are connected to each other via a bus 79 such as a system bus or a control bus such that various types of information can be exchanged.

The control unit 60 controls overall operations of the image processing apparatus 16. The control unit 60 is configured with a computer system including a CPU 60A, a ROM 60B, and a RAM 60C. Various programs, data, and the like for performing control by the CPU 60A are stored in advance in the ROM 60B. The RAM 60C temporarily stores various types of data.

The storage unit 62 is a non-volatile storage such as an HDD or an SSD. The storage unit 62 stores a program 63 for causing the control unit 60 to execute various types of processing, a machine-learned model 64 for performing shape restoration processing to be described later, a learning program 65 for causing the machine-learned model 64 to perform machine learning, and the like.

The display unit 70 is a display that displays a radiation image, various types of information, and the like. The operation unit 72 is used to allow a doctor to input an instruction for diagnosing a lesion of a breast using a radiation image, various types of information, and the like. The operation unit 72 includes, for example, various switches, a touch panel, a touch pen, a mouse, and the like.

The communication I/F 74 performs communication of various types of information between the console 12 and the PACS 14 via the network 17 by wireless communication or wired communication.

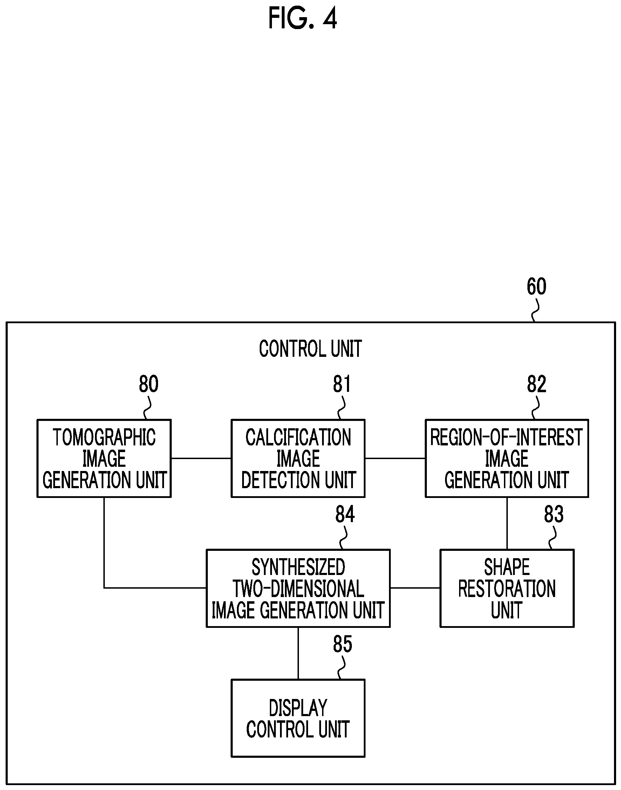

Fig. 4 illustrates an example of a function realized by the control unit 60 of the image processing apparatus 16. The CPU 60A of the control unit 60 realizes various functions by executing processing based on the program 63 stored in the storage unit 62. The control unit 60 functions as a tomographic image generation unit 80, a calcification image detection unit 81, a region-of-interest image generation unit 82, a shape restoration unit 83, a synthesized two-dimensional image generation unit 84, and a display control unit 85.

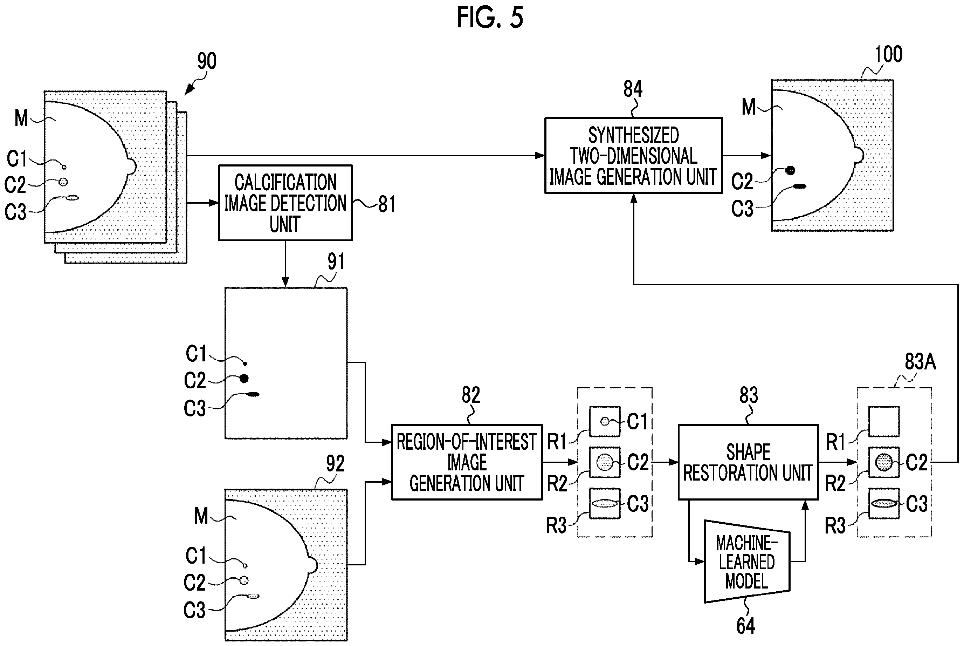

The tomographic image generation unit 80 has a function of generating a plurality of tomographic images 90 (refer to Fig. 5) from a series of the plurality of projection images. The tomographic image generation unit 80 acquires a series of the plurality of projection images from the console 12 of the mammography apparatus 10 or the PACS 14 based on an instruction for diagnosing a lesion. The tomographic image generation unit 80 generates a plurality of tomographic images 90 having different heights from the imaging surface 24A, from a series of the plurality of acquired projection images. For example, the tomographic image generation unit 80 generates a plurality of tomographic images 90 by reconfiguring a series of the plurality of projection images by a back projection method. As the back projection method, a filter back projection (FBP) method, a successive approximation reconfiguration method, or the like can be used. The tomographic image generation unit 80 outputs the plurality of generated tomographic images 90 to the calcification image detection unit 81 and the synthesized two-dimensional image generation unit 84.

Fig. 5 schematically illustrates a flow of processing by the image processing apparatus 16. Processing by the calcification image detection unit 81, the region-of-interest image generation unit 82, the shape restoration unit 83, and the synthesized two-dimensional image generation unit 84 will be described with reference to Fig. 5.

The calcification image detection unit 81 performs calcification image detection processing of detecting a tissue image in which an occurrence of calcification is expected in the breast M (hereinafter, calcification image) based on the plurality of tomographic images 90 generated by the tomographic image generation unit 80. For example, the calcification image detection unit 81 performs detection processing on each of the plurality of tomographic images 90 by using using a known computer-aided diagnosis (CAD) algorithm, and obtains a sum set of detection information (corresponding to a mask image 91 to be described later) obtained from each tomographic image 90. In the CAD algorithm, a probability (likelihood) indicating that a pixel in the tomographic image 90 is a calcification image is derived, and a pixel of which the probability is equal to or higher than a predetermined threshold value is detected as the calcification image.

The detection processing is not limited to the detection processing using the CAD algorithm (so-called rule-based detection processing). The calcification image detection unit 81 may perform detection processing using a machine-learned model obtained by performing machine learning. For example, the calcification image detection unit 81 may detect the calcification image by inputting the plurality of tomographic images 90 into the machine-learned model.

The detection result of the calcification image by the calcification image detection unit 81 is output as, for example, a mask image 91 representing a position of the calcification image. The mask image 91 is a binary image in which a pixel included in the calcification image is represented by "1" and the other pixels are represented by "0". The calcification image detection unit 81 outputs one mask image 91 for the plurality of tomographic images 90. By performing the detection processing using the plurality of tomographic images 90, the calcification image can be detected with high detection accuracy. In the example illustrated in Fig. 5, three calcification images C1 to C3 are detected by the calcification image detection unit 81.

The region-of-interest image generation unit 82 generates a region-of-interest image (hereinafter, referred to as a region-of-interest (ROI) image) based on one projection image 92 among a series of the plurality of projection images used for the reconfiguration processing by the tomographic image generation unit 80 and the detection result of the calcification image by the calcification image detection unit 81. The projection image 92 used for generation of the ROI image by the region-of-interest image generation unit 82 is a projection image obtained at a position facing the detection surface 20A of the radiation detector 20 among a series of the plurality of projection images. The position facing the detection surface 20A is a position at which the irradiation angle α is 0 degree. In the present embodiment, the position facing the detection surface 20A is the irradiation position P4 illustrated in Fig. 2.

Among a series of the plurality of projection images obtained at the plurality of irradiation positions P1 to P7, in the projection image 92 obtained at the irradiation position P4 facing the detection surface 20A, the shape of the calcification image is most accurately represented.

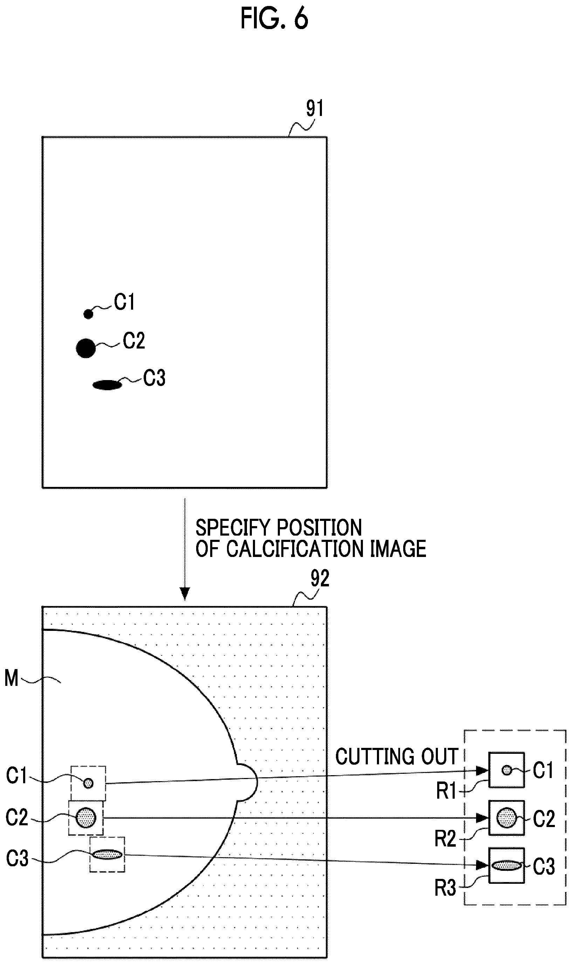

Fig. 6 conceptually illustrates an example of region-of-interest image generation processing by the region-of-interest image generation unit 82. The region-of-interest image generation unit 82 generates a ROI image by cutting out a region including the calcification image from the projection image 92 based on the mask image 91. In addition, in a case where the plurality of calcification images are detected in the calcification image detection processing, the region-of-interest image generation unit 82 individually generates a ROI image for each of the plurality of calcification images. In the example illustrated in Fig. 6, a ROI image is individually generated for each of three calcification images C1 to C3. Thereby, a ROI image R1 including the calcification image C1, a ROI image R2 including the calcification image C2, and a ROI image R3 including the calcification image C3 are generated.

The shape restoration unit 83 performs shape restoration processing of restoring a shape of the calcification image based on the ROI image generated by the region-of-interest image generation processing. In the present embodiment, the shape restoration unit 83 performs shape restoration processing by using the machine-learned model 64 obtained by performing machine learning on a relationship between the shape of the calcification image included in the ROI image and the shape of the calcification image obtained by the plain radiography. The shape restoration unit 83 inputs the ROI image to the machine-learned model 64, and acquires a restoration result 83A output from the machine-learned model 64. The shape restoration unit 83 outputs a restoration result 83A to the synthesized two-dimensional image generation unit 84. The machine-learned model 64 is, for example, a convolutional neural network (CNN) obtained by performing machine learning by deep learning.

The shape restoration processing performed by the shape restoration unit 83 is not limited to processing of changing the shape of the calcification image, and includes processing of making the calcification image that is unclear due to noise or the like clearer by removing the noise or the like. The projection image 92 includes a larger amount of information on a shape of the calcification image than the tomographic image 90. On the other hand, the projection image 92 is a radiation image obtained by tomosynthesis imaging with a low radiation dose, and thus includes a large amount of noise. The shape restoration unit 83 can make the calcification image that is unclear due to the influence of noise clear.

In addition, even in the calcification images detected by the calcification image detection unit 81, in a case where a noise or the like is erroneously detected as a calcification image, the erroneously-detected image is removed as a noise by the shape restoration processing. Thus, a shape of the erroneously-detected image is not restored. That is, the shape restoration processing reduces false positives in which a noise or the like is erroneously detected as a calcification image.

In the example illustrated in Fig. 5, the shape restoration unit 83 individually inputs the three ROI images R1 to R3 generated by the region-of-interest image generation processing to the machine-learned model 64. The ROI images R1 to R3 in which the shape of the calcification image is restored are output from the machine-learned model 64, as a restoration result 83A. In the example illustrated in Fig. 5, the calcification image C1 is an image obtained by erroneously detecting a noise as a calcification image by the calcification image detection unit 81. Thus, the calcification image C1 is removed together with a noise and the like by the shape restoration processing. Therefore, a shape of the calcification image C1 is not restored. On the other hand, shapes of the calcification images included in the ROI images R2 and R3 are restored by the shape restoration processing.

The synthesized two-dimensional image generation unit 84 performs synthesized two-dimensional image generation processing of generating a synthesized two-dimensional image 100 based on the shape restoration result 83A by the shape restoration processing and the plurality of tomographic images 90 generated by the tomographic image generation unit 80.

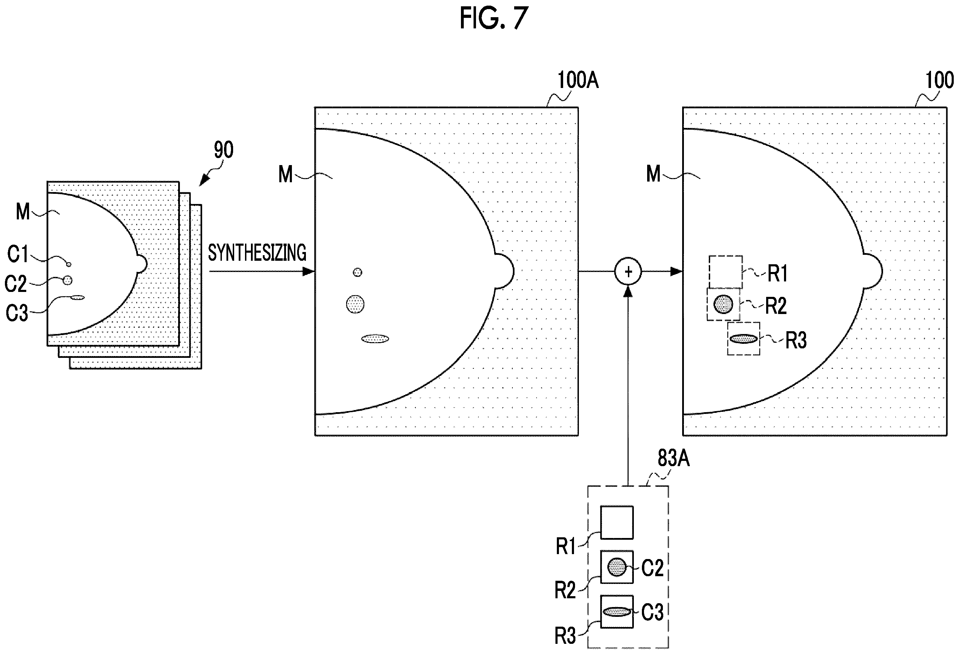

Fig. 7 conceptually illustrates an example of synthesized two-dimensional image generation processing by the synthesized two-dimensional image generation unit 84. First, the synthesized two-dimensional image generation unit 84 generates an image 100A corresponding to a simple two-dimensional image obtained by performing radiographic imaging at an irradiation position P4 facing the detection surface 20A, by synthesizing the plurality of tomographic images using an addition method, an averaging method, a maximum intensity projection method, a minimum intensity projection method, or the like. In addition, the synthesized two-dimensional image generation unit 84 generates a synthesized two-dimensional image 100 based on the image 100A and the restoration result 83A. For example, the synthesized two-dimensional image generation unit 84 generates a synthesized two-dimensional image 100 by replacing the regions corresponding to the ROI images R1 to R3 in the image 100A with the ROI images R1 to R3 included in the restoration result 83A. The synthesized two-dimensional image generation unit 84 may generate a synthesized two-dimensional image 100 by performing weighting addition of the ROI images R1 to R3 included in the restoration result 83A on the corresponding regions of the image 100A.

The display control unit 85 performs display processing of displaying the synthesized two-dimensional image 100 generated by the synthesized two-dimensional image generation processing on the display unit 70. The display control unit 85 may perform highlight display by changing a color or the like of the calcification image. In addition, the display control unit 85 may display one or more tomographic images 90 on the display unit 70 together with the synthesized two-dimensional image 100.

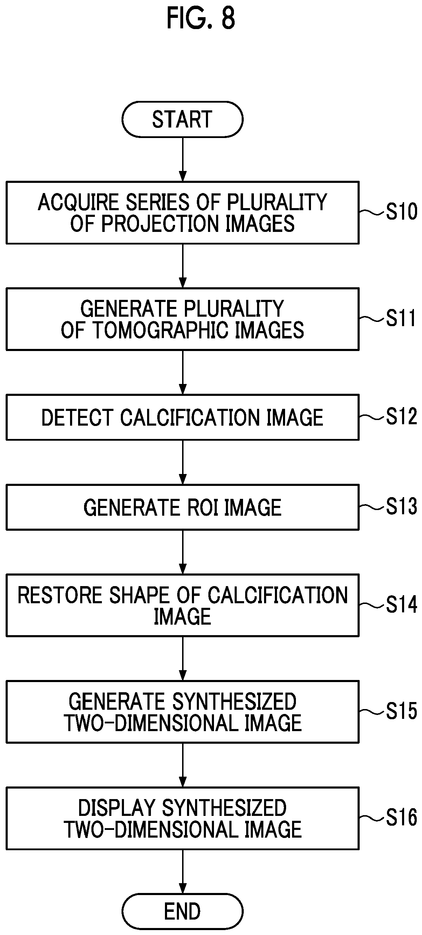

Next, a series of processing by the image processing apparatus 16 will be described with reference to Fig. 8. First, in step S 10, the tomographic image generation unit 80 acquires a series of a plurality of projection images from the console 12 of the mammography apparatus 10 or the PACS 14.

In step S 11, the tomographic image generation unit 80 generates a plurality of tomographic images 90 based on a series of the plurality of projection images acquired in step S10.

In step S12, the calcification image detection unit 81 detects a calcification image from the plurality of tomographic images 90 generated in step S 11, and generates a mask image 91 as a detection result.

In step S13, the region-of-interest image generation unit 82 generates a ROI image by cutting out a region including the calcification image from the projection image 92 obtained at a position facing the detection surface 20A of the radiation detector 20, by using the mask image 91 generated in step S12.

In step S14, the shape restoration unit 83 restores a shape of the calcification image based on the ROI image generated in step S13. Specifically, the shape restoration unit 83 inputs the ROI image to the machine-learned model 64, and acquires a restoration result 83A output from the machine-learned model 64.

In step S15, the synthesized two-dimensional image generation unit 84 generates a synthesized two-dimensional image 100 based on the restoration result 83A obtained in step S14 and the plurality of tomographic images 90 generated in step S11.

In step S16, the display control unit 85 displays the synthesized two-dimensional image 100 generated in step S15 on the display unit 70.

As described above, according to the technology of the present disclosure, the ROI image is generated from the projection image 92, which is obtained at the irradiation position P4 facing the detection surface 20A of the radiation detector 20, among the series of the plurality of projection images, and the synthesized two-dimensional image 100 is generated using a result obtained by restoring a shape of the calcification image based on the ROI image. Thereby, visibility of the shape of the calcification image in the synthesized two-dimensional image 100 is improved.

In particular, the projection image 92 obtained at the irradiation position P4 facing the detection surface 20A more accurately represents the shape of the calcification image, and thus, it is possible to accurately restore the shape of the calcification image by restoring the shape of the calcification image based on the ROI image generated from the projection image 92.

As described above, according to the technology of the present disclosure, it is possible to diagnose calcification of the breast M with high accuracy by using only the series of the plurality of projection images obtained by tomosynthesis imaging.

In the embodiment, the irradiation position P4 is a position facing the detection surface 20A (that is, a position at which α = 0). On the other hand, the irradiation position may not exist at the position facing the detection surface 20A. In such a case, the ROI image may be generated using the projection image obtained at the irradiation position closest to the position facing the detection surface 20A among the plurality of irradiation positions at which a series of the plurality of projection images are obtained.

Further, in the embodiment, the calcification image detection unit 81 detects the calcification image from the plurality of tomographic images 90. The calcification image detection unit 81 may detect only a calcification image (so-called pale calcification image) of which a signal value is equal to or smaller than a certain value. This is because a shape of the pale calcification image is not accurately represented and it is difficult to determine a type of the shape on the tomographic image 90 as a clinical image that is displayed on the display unit 70.

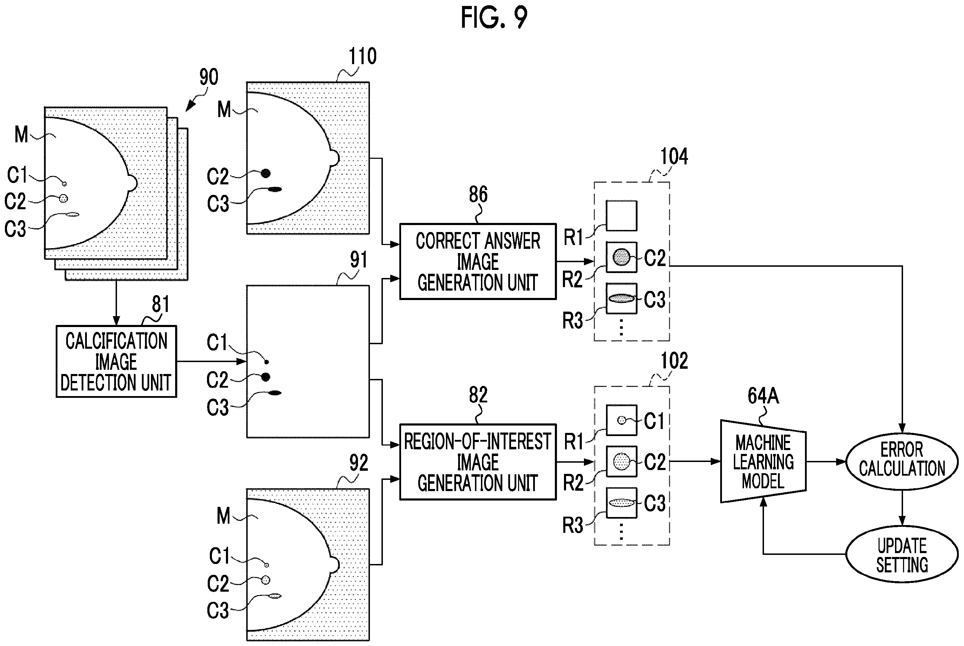

Next, a learning phase for generating the machine-learned model 64 will be described. Fig. 9 conceptually illustrates an example of learning processing in a learning phase. In a case where the CPU 60A of the control unit 60 executes the learning program 65 stored in the storage unit 62, learning processing illustrated in Fig. 9 is performed.

As illustrated in Fig. 9, in the learning phase, in order to generate training data including an input image 102 and a correct answer image 104, tomosynthesis imaging and plain radiography are performed on the same breast M. In the learning phase, the control unit 60 includes the tomographic image generation unit 80, the calcification image detection unit 81, the region-of-interest image generation unit 82, and a correct answer image generation unit 86, and these units function as a training data acquisition unit. The tomographic image generation unit 80, the calcification image detection unit 81, and the region-of-interest image generation unit 82 have the same configurations as the tomographic image generation unit 80, the calcification image detection unit 81, and the region-of-interest image generation unit 82 configured in the above-described operation phase.

The calcification image detection unit 81 detects a calcification image based on the plurality of tomographic images 90 generated by the tomographic image generation unit 80, and outputs a mask image 91. The region-of-interest image generation unit 82 generates a ROI image based on the projection image 92 obtained at the irradiation position P4 facing the detection surface 20A and the mask image 91. In the learning phase, the ROI image generated by the region-of-interest image generation unit 82 is set as an input image 102, and is used for learning of the machine learning model 64A.

The correct answer image generation unit 86 performs the same processing as the region-of-interest image generation unit 82 except that the projection image 110 obtained by plain radiography is used instead of the projection image 92 obtained by tomosynthesis imaging. That is, the correct answer image generation unit 86 generates a ROI image by cutting out a region including the calcification image from the projection image 110 based on the mask image 91. In the plain radiography, the radiation dose is higher than that in the tomosynthesis imaging, and thus, a clear calcification image that is less likely to be influenced by a noise is captured. In the learning phase, the ROI image generated by the correct answer image generation unit 86 is set as a correct answer image 104, and is used for learning of the machine learning model 64A.

In the example illustrated in Fig. 9, the calcification image C1 is an image obtained by erroneously detecting a noise as a calcification image by the calcification image detection unit 81. Therefore, the ROI image R1, which is generated by the correct answer image generation unit 86 based on the projection image 110 that is less likely to be influenced by a noise, does not include the calcification image C1.

The machine-learned model 64 is a neural network generated by performing, in a learning phase, machine learning on the machine learning model 64A using training data including the input image 102 and the correct answer image 104. The machine learning is performed on the machine learning model 64A using, for example, an error back propagation method. In the learning phase, error calculation between the determination result obtained by inputting the input image 102 to the machine learning model 64A and the correct answer image 104 and update setting of weights and biases are repeatedly performed. The machine learning model 64A obtained by performing machine learning in the learning phase is stored in the storage unit 62, as a machine-learned model 64. The machine learning of the machine learning model 64A may be performed in the image processing apparatus 16 or in an external apparatus.

The machine-learned model 64 obtained by performing machine learning in this way restores the shape of the calcification image included in the ROI image generated by the region-of-interest image generation unit 82 into the shape of the calcification image included in the ROI image generated by the correct answer image generation unit 86.

Hereinafter, various modification examples of the embodiment will be described.

### [First Modification Example]

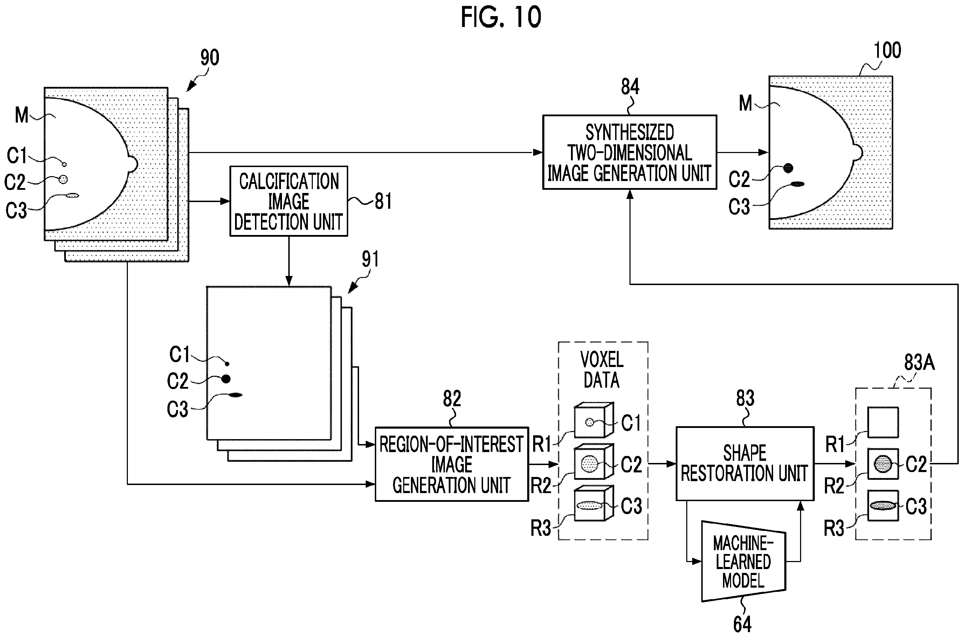

Fig. 10 illustrates a function realized by the control unit 60 of the image processing apparatus 16 according to a first modification example. In the first modification example, the calcification image detection processing by the calcification image detection unit 81 and the region-of-interest image generation processing by the region-of-interest image generation unit 82 are different from those in the embodiment.

In the present modification example, the calcification image detection unit 81 outputs the plurality of mask images 91 corresponding to each of the plurality of tomographic images 90.

In the present modification example, the region-of-interest image generation unit 82 generates a ROI image based on the plurality of tomographic images 90 generated by the tomographic image generation unit 80 and the plurality of mask images 91. Specifically, the region-of-interest image generation unit 82 generates a ROI image by cutting out a region including the calcification image from the plurality of tomographic images 90 based on the plurality of mask images 91. In the present modification example, the ROI image generated by the region-of-interest image generation unit 82 is voxel data represented by units of voxels in a three-dimensional space. The ROI images generated by the region-of-interest image generation unit 82 are cut out from the plurality of tomographic images 90. Thus, an amount of information of the shape of the calcification image is reduced as compared with the case of using the projection images, but there is an advantage that a noise is reduced.

In the present modification example, the ROI image as voxel data is input to the shape restoration unit 83. The shape restoration unit 83 inputs the ROI image input from the region-of-interest image generation unit 82 to the machine-learned model 64, and acquires a restoration result 83A output from the machine-learned model 64. The restoration result 83A includes a two-dimensional ROI image including the calcification image of which a shape is restored. In the example illustrated in Fig. 10, similarly to the embodiment, the calcification image C1 is removed together with a noise and the like by the shape restoration processing. Therefore, a shape of the calcification image C1 is not restored.

The synthesized two-dimensional image generation unit 84 generates a synthesized two-dimensional image 100 based on the restoration result 83A and the plurality of tomographic images 90 generated by the tomographic image generation unit 80, similarly to the embodiment.

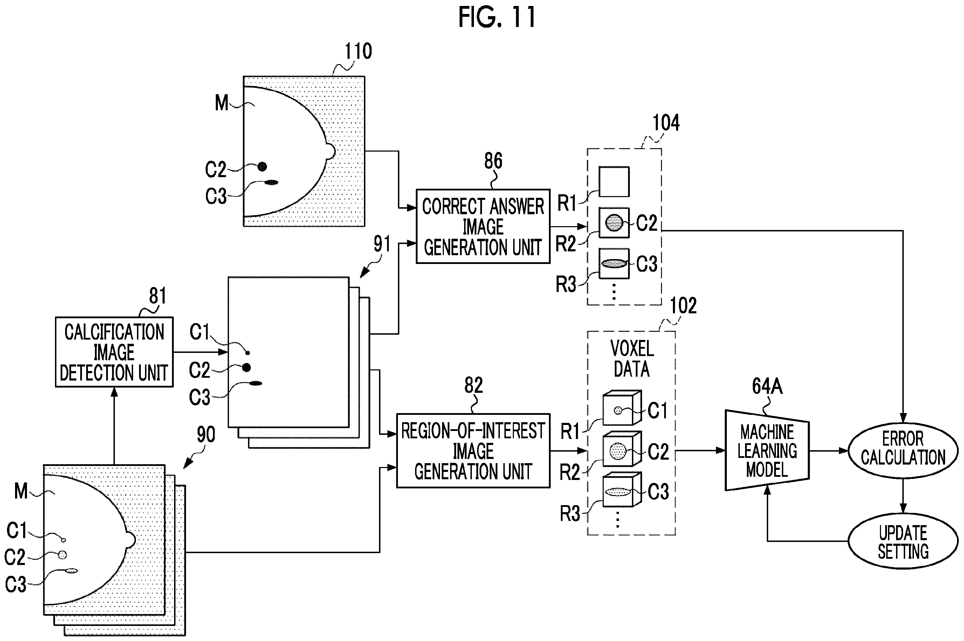

Fig. 11 conceptually illustrates a learning process according to the first modification example. The learning processing according to the present modification example is different from the learning processing according to the embodiment only in that, in order to generate the input image 102, the region-of-interest image generation unit 82 generates a ROI image based on the plurality of tomographic images 90 and the plurality of mask images 91. In the present modification example, the ROI image as voxel data is set as the input image 102, and is used for learning of the machine learning model 64A. The correct answer image 104 is the same as that in the embodiment.

In the present modification example, the machine-learned model 64 restores the shape of the calcification image included in the ROI image generated as voxel data by the region-of-interest image generation unit 82 into the shape of the calcification image included in the ROI image generated by the correct answer image generation unit 86.

### [Other Modification Examples]

In the embodiment and the modification example, in the learning phase, the input image 102 and the correct answer image 104 are generated based on the radiation images obtained by performing tomosynthesis imaging and plain radiography on the breast M as a subject. Alternatively, the input image 102 and the correct answer image 104 may be generated based on radiation images obtained by performing tomosynthesis imaging and plain radiography on a phantom (for example, a breast phantom) as a subject.

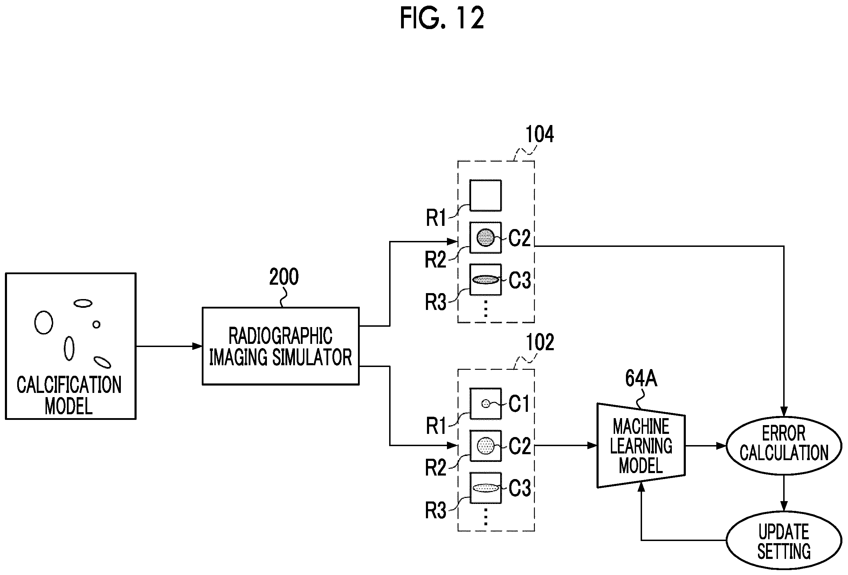

Further, the input image 102 and the correct answer image 104 may be generated by using simulations by a computer. For example, as illustrated in Fig. 12, a radiographic imaging simulator 200 generates the input image 102 and the correct answer image 104 based on a calcification model.

That is, the machine-learned model 64 may be a neural network obtained by performing machine learning using the input image 102 and the correct answer image 104, which are generated by a simulation or by imaging using a phantom. In a case where the training data is created by radiographic imaging using the breast M as a subject, there is a problem of exposure to radiation. However, by using a simulation or a phantom, a large amount of training data can be generated in a pseudo manner, and thus machine learning can be performed on the machine learning model 64A.

The embodiment and the modification examples can be appropriately combined as long as there is no contradiction.

In addition, in the embodiment and the modification examples, as a hardware structure of a processing unit that executes various processing, such as the tomographic image generation unit 80, the calcification image detection unit 81, the region-of-interest image generation unit 82, the shape restoration unit 83, the synthesized two-dimensional image generation unit 84, the display control unit 85, and the correct answer image generation unit 86, for example, the following various processors can be used. The various processors include a graphics processing unit (GPU) in addition to a CPU. In addition, the various processors are not limited to a general-purpose processor such as a CPU that functions as various processing units by executing software (program), and include a dedicated electric circuit, which is a processor having a circuit configuration specifically designed to execute specific processing, such as a programmable logic device (PLD) or an application specific integrated circuit (ASIC) that is a processor of which the circuit configuration may be changed after manufacturing such as a field programmable gate array (FPGA).

One processing unit may be configured by one of the various processors, or may be configured by a combination of the same or different kinds of two or more processors (for example, a combination of a plurality of FPGAs or a combination of the CPU and the FPGA). In addition, a plurality of processing units may be configured by one processor.

As an example in which the plurality of processing units are configured by one processor, firstly, as represented by a computer such as a client and a server, a form in which one processor is configured by a combination of one or more CPUs and software and the processor functions as the plurality of processing units may be adopted. Secondly, as represented by a system on chip (SoC) or the like, a form in which a processor that realizes the function of the entire system including the plurality of processing units by one integrated circuit (IC) chip is used may be adopted. As described above, the various processing units are configured by using one or more various processors as a hardware structure.

Furthermore, as the hardware structure of the various processors, more specifically, an electrical circuit (circuitry) in which circuit elements such as semiconductor elements are combined can be used.

In addition, in the embodiment and the modification examples, a form in which the program 63 is stored in the storage unit 62 in advance has been described. On the other hand, the present invention is not limited thereto. The program 63 may be provided by being recorded in a non-transitory recording medium such as a compact disc read only memory (CD-ROM), a digital versatile disc read only memory (DVD-ROM), or a Universal Serial Bus (USB) memory. Further, the program 63 may be downloaded from an external apparatus via a network.

The described contents and the illustrated contents are the detailed description of the parts according to the technology of the present disclosure, and are merely examples of the technology of the present disclosure. For example, the descriptions related to the configuration, the function, the operation, and the effect are descriptions related to examples of a configuration, a function, an operation, and an effect of a part according to the technology of the present disclosure. Therefore, it goes without saying that, in the described contents and illustrated contents, unnecessary parts may be deleted, new components may be added, or replacements may be made without departing from the spirit of the technology of the present disclosure. Further, in order to avoid complications and facilitate understanding of the part according to the technology of the present disclosure, in the described contents and illustrated contents, descriptions of technical knowledge and the like that do not require particular explanations to enable implementation of the technology of the present disclosure are omitted.

All documents, patent applications, and technical standards mentioned in this specification are incorporated herein by reference to the same extent as in a case where each document, each patent application, and each technical standard are specifically and individually described by being incorporated by reference.

## Claims

1. An image processing apparatus comprising:
at least one processor,
wherein the processor is configured to execute:
calcification image detection processing of detecting a calcification image based on a plurality of tomographic images obtained from a series of a plurality of projection images obtained by tomosynthesis imaging of a breast;
region-of-interest image generation processing of generating a region-of-interest image based on a detection result by the calcification image detection processing by cutting out a region including the calcification image, from a projection image obtained at an irradiation position among the plurality of projection images or the plurality of tomographic images, the irradiation position being closest to a position facing a detection surface of a radiation detector;
shape restoration processing of restoring a shape of the calcification image based on the region-of-interest image generated by the region-of-interest image generation processing; and
synthesized two-dimensional image generation processing of generating a synthesized two-dimensional image based on a shape restoration result by the shape restoration processing and the plurality of tomographic images.

2. The image processing apparatus according to claim 1,
wherein the processor is configured to:
in a case where a plurality of the calcification images are detected in the calcification image detection processing, individually generate the region-of-interest image for each of the plurality of calcification images in the region-of-interest image generation processing.

3. The image processing apparatus according to claim 1 or 2,
wherein the processor is configured to:
execute the shape restoration processing by inputting the region-of-interest image into a machine-learned model.

4. The image processing apparatus according to claim 3,
wherein the machine-learned model is a neural network obtained by performing machine learning by using, as an input image, the region-of-interest image and using, as a correct answer image, an image generated by cutting out a region including the calcification image from a projection image obtained by plain radiography in which radiation is emitted from a position facing a detection surface of a radiation detector.

5. The image processing apparatus according to claim 3,
wherein the machine-learned model is a neural network obtained by performing machine learning by using an input image and a correct answer image that are generated by a simulation or by imaging using a phantom.

6. An image processing method comprising:
a calcification image detection step of detecting a calcification image based on a plurality of tomographic images obtained from a series of a plurality of projection images obtained by tomosynthesis imaging of a breast;
a region-of-interest image generation step of generating a region-of-interest image based on a detection result by the calcification image detection step by cutting out a region including the calcification image, from a projection image obtained at an irradiation position among the plurality of projection images or the plurality of tomographic images, the irradiation position being closest to a position facing a detection surface of a radiation detector;
a shape restoration step of restoring a shape of the calcification image based on the region-of-interest image generated by the region-of-interest image generation step; and
a synthesized two-dimensional image generation step of generating a synthesized two-dimensional image based on a shape restoration result by the shape restoration step and the plurality of tomographic images.

7. A program causing a computer to execute:
calcification image detection processing of detecting a calcification image based on a plurality of tomographic images obtained from a series of a plurality of projection images obtained by tomosynthesis imaging of a breast;
region-of-interest image generation processing of generating a region-of-interest image based on a detection result by the calcification image detection processing by cutting out a region including the calcification image, from a projection image obtained at an irradiation position among the plurality of projection images or the plurality of tomographic images, the irradiation position being closest to a position facing a detection surface of a radiation detector;
shape restoration processing of restoring a shape of the calcification image based on the region-of-interest image generated by the region-of-interest image generation processing; and
synthesized two-dimensional image generation processing of generating a synthesized two-dimensional image based on a shape restoration result by the shape restoration processing and the plurality of tomographic images.

8. A machine learning method comprising:
detecting a calcification image based on a plurality of tomographic images obtained from a series of a plurality of projection images obtained by tomosynthesis imaging of a breast;
setting, as an input image, a region-of-interest image generated by cutting out a region including the calcification image, from a projection image obtained at an irradiation position among the plurality of projection images or the plurality of tomographic images, the irradiation position being closest to a position facing a detection surface of a radiation detector; and
performing machine learning on a neural network by using, as a correct answer image, an image generated by cutting out a region including the calcification image from a projection image obtained by plain radiography in which radiation is emitted from a position facing a detection surface of a radiation detector.
